(19) **Europäisches Patentamt / European Patent Office / Office européen des brevets**

(11) **EP 2 923 677 B1**

(12) **FASCICULE DE BREVET EUROPEEN**

(45) Date de publication et mention de la délivrance du brevet:
**09.11.2016 Bulletin 2016/45**

(51) Int Cl.:
***A61F 2/46*** *(2006.01)*  ***A61B 17/92*** *(2006.01)*

(21) Numéro de dépôt: **15160422.0**

(22) Date de dépôt: **23.03.2015**

(54) **DISPOSITIF D'ASSISTANCE À LA MISE EN PLACE D'UN INSTRUMENT ORTHOPÉDIQUE**

HILFSVORRICHTUNG ZUM ANBRINGEN EINES ORTHOPÄDISCHEN INSTRUMENTS

DEVICE FOR ASSISTING WITH THE PLACEMENT OF AN ORTHOPAEDIC INSTRUMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.03.2014 FR 1452677**

(43) Date de publication de la demande:
**30.09.2015 Bulletin 2015/40**

(73) Titulaire: **CENTRE NATIONAL DE LA RECHERCHE SCIENTIFIQUE -CNRS- 75016 Paris (FR)**

(72) Inventeurs:
• **Haiat, Guillaume**
**94410 Saint Maurice (FR)**
• **Mathieu, Vincent**
**75013 Paris (FR)**
• **Michel, Adrien**
**94110 Arcueil (FR)**

(74) Mandataire: **Gendron, Vincent Christian et al Fédit-Loriot**
**38, avenue Hoche**
**75008 Paris (FR)**

(56) Documents cités:
**WO-A1-2009/105817     WO-A2-2007/070287**
**DE-A1-102006 030 343     US-A1- 2005 101 962**

## Description

L'invention concerne un dispositif d'assistance à la mise en place d'un instrument orthopédique, destiné à être inséré au niveau d'un os récepteur par impaction au moyen d'un outil de percussion, ou impacteur, apte à appliquer une force d'impaction audit instrument orthopédique.

Sous le terme d'instrument orthopédique, on désigne l'ensemble des implants pour prothèses orthopédiques destinés à être implantés au niveau du tissu osseux afin de procéder au remplacement des articulations déficientes, mais également les dispositifs annexes utilisés pour préparer l'os récepteur afin de permettre la pose de l'implant définitif et, en particulier, les râpes chirurgicales d'implant orthopédique. Parmi les prothèses orthopédiques, les implants disponibles sont multiples. On peut notamment distinguer les prothèses totales de hanche, les prothèses de genoux, les prothèses d'épaule ou encore les prothèses de coude de cheville ou de rachis.

Un domaine d'application de la présente invention concerne principalement, mais non exclusivement, la mise en place d'instruments orthopédiques pour des prothèses de hanche. La grande majorité des modèles proposés de prothèse de hanche ont en commun une première partie, constituée d'une tige fémorale destiné à être implantée dans le canal médullaire du fémur et d'une tête prothétique formée d'une pièce sphérique éventuellement démontable se substituant à la tête du fémur, et une seconde partie, constituée d'un cotyle prothétique, destiné à être implanté dans la cavité cotyloïde située à la face latérale de l'os iliaque. Le cotyle prothétique, qui est donc destiné à remplacer la partie articulaire du bassin, est plus précisément constitué d'une cupule cotyloïdienne, qui est une pièce approximativement hémisphérique, généralement en métal, implanté dans l'os du bassin et dans laquelle se place un insert avec lequel s'articule la tête prothétique.

En particulier, la mise en place du cotyle prothétique dans la cavité osseuse peut être réalisée par simple impaction, au moyen d'un ancillaire de pose prévu à cet effet, par l'intermédiaire duquel le cotyle prothétique est impacté par le chirurgien, à l'aide d'un impacteur, typiquement un marteau, venant frapper l'ancillaire de pose. Un exemple d'ancillaire de pose du cotyle prothétique consiste en un manche rigide de manipulation, pourvu dans sa partie distale, d'une tête de préhension, permettant la préhension puis le positionnement du cotyle jusqu'à l'emplacement d'impaction du cotyle dans la cavité osseuse de la hanche et, dans sa partie proximale, d'une surface d'impact destinée à être impactée au moyen d'une face de frappe de l'impacteur prévue à cet effet pour appliquer la force d'impaction nécessaire à la mise en place du cotyle prothétique dans la cavité osseuse.

La réussite chirurgicale lors de la mise en place du cotyle prothétique et, plus généralement, d'un implant de prothèse orthopédique destiné à être implanté par impaction, repose sur un degré d'insertion suffisant de cet implant dans l'os récepteur, dont la stabilité biomécanique va dépendre. Cette stabilité biomécanique est en réalité déterminée par deux phénomènes distincts :

- la stabilité primaire, qui correspond au degré d'attachement ou d'ancrage de l'élément de prothèse dans l'os juste après l'opération (stabilité post-opératoire immédiate), et qui dépend de la qualité osseuse et de la procédure chirurgicale, qui sera détaillée plus bas ;
- la stabilité secondaire, qui correspond à la repousse osseuse par l'intermédiaire des phénomènes d'ostéointégration, et qui dépend de la stabilité primaire et de la capacité de l'os à cicatriser.

Suivant la procédure chirurgicale classique, le chirurgien procède à un forage dans l'os du bassin, de manière à réaliser une cavité hémisphérique permettant une tenue de l'implant, tout en stimulant les phénomènes de remodelage osseux, ce qui permet d'améliorer les phénomènes d'ostéointégration durant la phase de cicatrisation. Une fois la cavité réalisée, le chirurgien insère le cotyle prothétique en force dans la cavité par impaction, à l'aide de l'impacteur et de l'ancillaire de pose, jusqu'à ce qu'il juge qu'un degré d'insertion suffisant de l'implant est atteint de façon à obtenir une bonne stabilité primaire de l'implant et ce, sans engendrer de fractures dans l'os. La stabilité primaire est en effet un élément essentiel pour la réussite chirurgicale. Typiquement, une bonne stabilité primaire correspond à une situation de pose dans laquelle le degré d'insertion de l'implant est optimal, se traduisant par un maximum de surface de contact entre l'os et l'implant. Cependant, il est aujourd'hui difficile pour le chirurgien de pouvoir quantifier finement le nombre et la force des impacts à générer au moyen de l'impacteur sur l'ancillaire de pose, qui sont nécessaires à l'obtention d'une bonne stabilité primaire, sans endommager pour autant l'os récepteur. Un compromis doit ainsi être trouvé entre une énergie d'impact suffisamment forte, de façon à obtenir une bonne stabilité primaire, qui correspond à un maximum de surface de contact entre l'os et l'implant, et une énergie d'impact suffisamment faible, de façon à ne pas risquer de fractures per-opératoire de l'os récepteur.

Or, aujourd'hui, seules des méthodes empiriques sont utilisées. Typiquement, durant l'opération, les chirurgiens se fondent sur leur expérience et, en particulier, sur le bruit de l'impact afin de déterminer s'ils doivent frapper plus ou moins fort et/ou continuer d'appliquer des coups de marteau. Toutefois, en utilisant ce type de méthode, les chirurgiens ne peuvent pas se prévaloir de la certitude absolue que le degré d'insertion de l'implant dans l'os récepteur soit optimal. En outre, un des inconvénients résidant dans l'application de cette méthode est que lorsque l'implant est insuffisamment inséré dans l'os, il peut s'ensuivre une désolidarisation de ce dernier à son support. Cela peut se traduire par des lésions importantes de la cavité creusée au niveau de l'os récepteur. Dans ce cas, le chirurgien devra alors par exemple procéder à une nouvelle intervention afin d'élargir la cavité osseuse, ce qui permettra ensuite le positionnement d'un nouvel implant ayant des dimensions adaptées.

On connaît du document de brevet EP1433445, une méthode de mesure per-opératoire de la stabilité mécanique d'une prothèse orthopédique destinée à être implantée dans un os par insertion forcée, destinée à aider le chirurgien à estimer la stabilité implantaire. Cette méthode consiste à mesurer un glissement relatif de la prothèse par rapport à l'os récepteur sous l'action d'une charge d'essai opportunément prédéterminée, appliquée à la prothèse ou à l'os récepteur. Cette méthode repose toutefois sur une mise en oeuvre complexe.

Par ailleurs, ce besoin identifié de pouvoir évaluer finement le nombre d'impacts nécessaire pour atteindre un degré d'insertion suffisant sans risquer de provoquer de fractures per-opératoire de l'os récepteur, se retrouve également lors de la mise en oeuvre de la procédure chirurgicale consistant à impacter une râpe chirurgicale d'implant orthopédique, en particulier une râpe fémorale pour prothèse de hanche. En effet, ce type de râpe est classiquement utilisée afin de réaliser un trou dans le canal médullaire du fémur en "fraisant" l'os de façon d'une part, à préparer la forme du canal pour que l'implant s'y adapte parfaitement et, d'autre part, à stimuler la repousse osseuse en enlevant du tissu osseux superficiellement. Ces râpes orthopédiques ne sont pas un élément de l'implant (tel que le cotyle ou la tige fémorale) puisqu'on les enlève après les avoir impactées, de façon à permettre la pose de l'implant définitif. Cependant, le principe de mise en place est similaire dans la mesure où il consiste à impacter directement la râpe avec l'impacteur (sans l'intermédiaire d'un ancillaire) jusqu'à atteindre un degré d'insertion suffisant en prenant soin de ne pas provoquer de fracture de l'os récepteur dans lequel la râpe est insérée.

L'art antérieur le plus proche est divulgué par US 2005/101962.

Dans ce contexte, un but de la présente invention est de proposer un dispositif d'assistance à la mise en place d'un instrument orthopédique dans un os récepteur, qui permettent de fournir une évaluation précise du degré d'insertion de l'instrument orthopédique dans l'os récepteur durant l'opération, offrant au chirurgien un véritable outil d'aide à la décision capable de le renseigner en temps réel notamment sur la stabilité primaire d'implants orthopédiques, tout en étant simple d'utilisation et de mise en oeuvre.

A cet effet, l'invention concerne un dispositif d'assistance à la mise en place d'un instrument orthopédique destiné à être inséré au niveau d'un os récepteur par impaction, ledit dispositif comprenant un impacteur comportant au moins une surface de frappe adaptée pour venir impacter une surface d'impact couplée audit instrument orthopédique pour appliquer une force d'impaction audit instrument orthopédique. Selon l'invention, le dispositif comprend un capteur de force apte à mesurer, lors de chaque impact, ladite force d'impaction appliquée par l'impacteur sur ladite surface d'impact et à fournir un signal de mesure indicatif de la variation temporelle de ladite force d'impaction appliquée, ledit capteur de force étant relié à des moyens de traitement conçus pour déterminer, à partir de la variation temporelle de ladite force d'impaction fournie pour chaque impact successivement, un indicateur représentatif du degré d'insertion dudit instrument orthopédique dans l'os récepteur.

L'invention repose donc sur la mise en oeuvre d'un simple capteur de force, délivrant des signaux de mesure aptes à traduire la variation temporelle de la force appliquée pour chaque impact successivement, dont l'enregistrement et l'analyse vont permettre de déterminer un indicateur révélateur de la rigidité du contact os-instrument orthopédique et partant, du degré d'insertion de l'instrument orthopédique dans l'os récepteur. Le dispositif de l'invention permet donc de renseigner, en temps réel, le chirurgien sur le degré d'insertion atteint au cours de l'intervention d'insertion de l'instrument orthopédique dans l'os récepteur. Au surplus, outre son coût réduit, le dispositif de l'invention est particulièrement simple d'utilisation et, avantageusement, ne modifie en aucune façon le mode opératoire du chirurgien.

Avantageusement, ledit indicateur est déterminé par lesdits moyens de traitement comme étant la moyenne de ladite force d'impaction appliquée, calculée sur une fenêtre temporelle de durée programmable positionnée sur ledit signal de mesure, l'instant de début de ladite fenêtre temporelle étant définie par rapport à un instant correspondant à l'amplitude maximale dudit signal de mesure.

Il a pu être montré que l'indicateur ainsi construit est corrélé à la force d'arrachement, c'est-à-dire à la force nécessaire à appliquer pour arracher l'instrument orthopédique de son ancrage dans l'os récepteur. Dit autrement, l'indicateur ainsi construit fournit une évaluation de la force d'arrachement, qui constitue un facteur déterminant de la stabilité implantaire.

Selon un premier mode de réalisation, l'instant de début de ladite fenêtre temporelle coïncide avec l'instant correspondant à l'amplitude maximale dudit signal de mesure.

De préférence, selon ce premier mode de réalisation, ladite fenêtre temporelle a une durée comprise entre 1,2 ms et 2 ms, avantageusement entre 1,4 ms et 1,8 ms et avantageusement encore égale à 1,6 ms.

Selon un deuxième mode de réalisation, l'instant de début de ladite fenêtre temporelle est défini avec un retard prédéterminé par rapport à l'instant correspondant à l'amplitude maximale dudit signal de mesure.

De préférence, selon ce deuxième mode de réalisation, ladite fenêtre temporelle a une durée prédéfinie inférieure à 10 ms.

De préférence, ledit indicateur est calculé de la façon suivante :

$$I_1 = \frac{1}{A_1 . (t_2 - t_1)} \int_{t_1}^{t_2} A(t).dt$$

où :

A(t) correspond à l'amplitude dudit signal de mesure à l'instant t ;

$A_1$ correspond à l'amplitude maximale dudit signal de mesure ; et

$t_1$ et $t_2$ correspondent respectivement aux instants de début et de fin de ladite fenêtre temporelle.

En variante, ledit indicateur est déterminé par lesdits moyens de traitement comme étant la durée d'impact mesurée à partir de ladite variation temporelle.

Avantageusement lesdits moyens de traitement comprennent des moyens de contrôle de la convergence dudit indicateur vers une valeur stationnaire dudit indicateur en fonction du nombre d'impacts de manière à évaluer ledit degré d'insertion dudit instrument orthopédique dans l'os récepteur.

Selon un mode de réalisation, lesdits moyens de traitement de l'invention sont conçus pour détecter lorsque ledit indicateur atteint un palier de saturation, indiquant que ledit degré d'insertion est suffisant. En effet, l'atteinte par l'indicateur d'un palier sensiblement stable à partir d'un certain nombre d'impacts, signifie que la rigidité du contact entre l'instrument orthopédique et l'os récepteur ne varie plus, moyennant quoi le degré d'insertion est considéré comme optimal ou, en tous les cas, comme suffisant.

En variante, les moyens de traitement peuvent également comparer la valeur de l'indicateur à une valeur seuil définie à l'aide d'abaques.

Avantageusement, lesdits moyens de traitement sont couplés à des moyens d'alerte sonore et/ou visuelle aptes à fournir une indication visuelle et/ou sonore lorsque le degré d'insertion dudit instrument orthopédique dans l'os récepteur évalué par lesdits moyens de traitement est suffisant.

Ledit instrument orthopédique peut être constitué d'un implant pour prothèse orthopédique, ledit impacteur agissant alors pour impacter ledit implant en coopération avec un ancillaire de pose consistant en un manche rigide de manipulation, pourvu dans sa partie distale, d'une tête de préhension dudit implant et, dans sa partie proximale, de ladite surface d'impact.

Ledit instrument orthopédique peut également être constitué d'une râpe chirurgicale d'implant orthopédique, notamment une râpe fémorale pour prothèse de hanche, comportant une partie de préhension pourvue à son extrémité supérieure de ladite surface d'impact.

Dans un mode de réalisation, ledit capteur de force peut être monté sur ladite surface de frappe dudit impacteur.

Dans un autre mode de réalisation, ledit capteur de force peut être monté sur ladite surface d'impact.

Selon un mode de réalisation, ledit capteur de force peut être constitué de jauges de déformations.

En variante, ledit capteur de force peut être constitué d'un accéléromètre.

Une méthode (non revendiquéé) de traitement d'un signal représentatif d'une force d'impaction appliquée au moyen d'une surface de frappe d'un impacteur sur une surface d'impact couplée à un instrument orthopédique destiné à être inséré au niveau d'un os récepteur par impaction consiste en un procédé comprenant des étapes dans lesquelles :

on recueille, au moyen d'un capteur de force équipant ladite surface de frappe dudit impacteur, un signal de mesure indicatif de la variation temporelle de ladite force d'impaction appliquée par l'impacteur sur ladite surface d'impact lors de chaque impact, on détermine, à partir de la variation temporelle de ladite force d'impaction fournie pour chaque impact successivement, un indicateur représentatif d'un degré d'insertion dudit instrument orthopédique dans l'os récepteur.

La méthode comprend avantageusement une étape de contrôle de l'évolution dudit indicateur en fonction du nombre d'impacts dans laquelle on surveille la convergence dudit indicateur vers une valeur stationnaire dudit indicateur en fonction du nombre d'impacts de manière à évaluer ledit degré d'insertion dudit instrument orthopédique dans l'os récepteur.

La méthode comprend préférentiellement une étape de commande de moyens d'alerte sonore et/ou visuelle lorsque ledit indicateur atteint ladite valeur stationnaire ou une valeur seuil définie préalablement par des abaques.

La méthode décrite ci-dessus peut être mise en oeuvre par des moyens numériques de traitement, par exemple un microprocesseur, un microcontrôleur ou autre.

D'autres caractéristiques et avantages de l'invention ressortiront clairement de la description qui en est faite ci-après d'un mode de réalisation particulier de l'invention présenté à titre indicatif et nullement limitatif, en référence aux dessins annexés, dans lesquels:

- la Figure 1 est une représentation schématique d'un dispositif d'assistance à la mise en place d'un implant de prothèse orthopédique conforme à l'invention;
- la Figure 2 est un schéma illustrant un exemple de six signaux de mesure correspondant à la variation de la force appliquée sur l'ancillaire de pose en fonction du temps au cours de différents impacts et illustrant le calcul d'un indicateur représentatif du degré d'insertion de l'implant dans l'os récepteur correspondant à la moyenne du signal calculée sur une fenêtre temporelle d'intégration prédéfinie ;

- la Figure 3 est un schéma illustrant un autre mode de réalisation pour le positionnement de la fenêtre temporelle d'intégration utilisée pour calculer l'indicateur selon l'invention ;
- la Figure 4 est un schéma illustrant un exemple de deux signaux de mesure correspondant à la variation de la force appliquée sur l'ancillaire de pose en fonction du temps au cours de différents impacts et illustrant le calcul de l'indicateur représentatif du degré d'insertion de l'implant dans l'os récepteur selon une variante de réalisation ;
- la Figure 5 est un schéma illustrant la variation de l'indicateur selon l'une ou l'autre des deux variantes en fonction du nombre d'impacts.

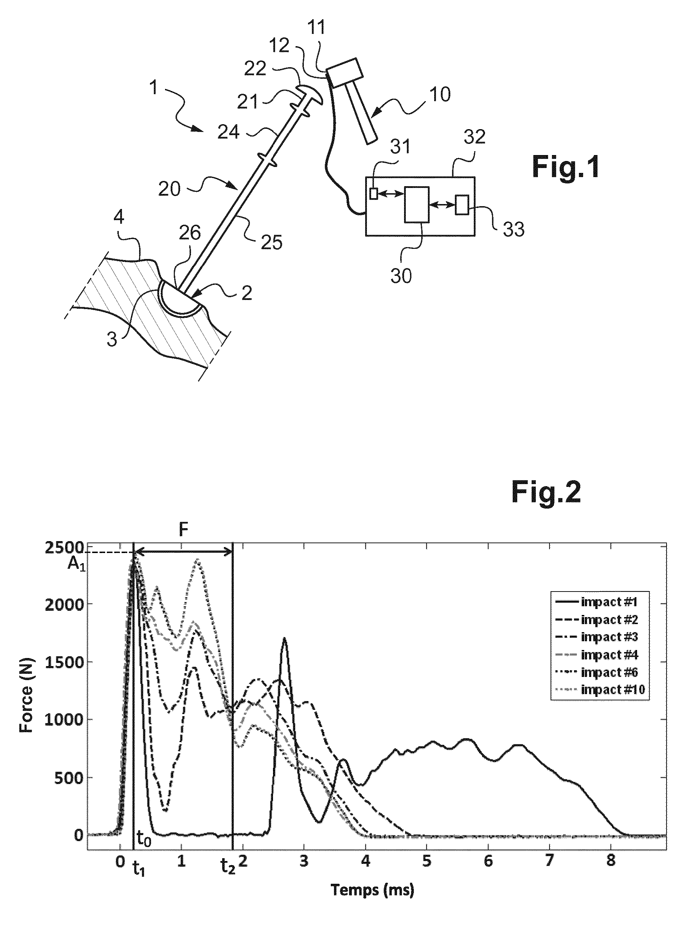

On a représenté sur la figure 1 un dispositif 1 d'assistance à la mise en place d'un instrument orthopédique 2, qui est constitué dans cet exemple d'un implant pour prothèse orthopédique et en particulier d'un cotyle prothétique 2, destiné à être inséré progressivement par impaction dans un os récepteur 3, formé par une cavité osseuse de la hanche 4 du patient. Le dispositif 1 comprend un outil de percussion ou impacteur 10, de type marteau ou équivalent, utilisé par le chirurgien pour produire un impact sur le cotyle prothétique 2 par l'intermédiaire d'un ancillaire de pose 20 du cotyle prothétique 2. L'ancillaire de pose 20 comprend essentiellement un manche comportant une tige rigide 21 à l'extrémité proximale de laquelle est fixé rigidement un pommeau présentant une surface 22 sensiblement convexe, formant une surface d'impact. En parcourant la tige 21 depuis son extrémité proximale vers son extrémité distale, la tige 21 comprend encore une poignée de manipulation 24 solidaire de la tige 21, puis un tronçon 25 se terminant par une tête 26 de préhension du cotyle prothétique 2, non illustrée en détail.

L'impacteur 10 comporte quant à lui une surface de frappe 11 destinée à venir impacter la surface d'impact 22 de l'ancillaire de pose 20, pour appliquer la force d'impaction nécessaire à la mise en place de l'implant 2 dans la cavité osseuse 3. Plusieurs impacts de sensiblement la même intensité sont préférentiellement réalisés au moyen de l'impacteur de façon à obtenir un enfoncement progressif de l'instrument orthopédique, en l'occurrence le cotyle prothétique, dans l'os récepteur. En outre, la direction de la force d'impaction est sensiblement alignée le long de l'axe de l'implant.

Comme expliqué plus haut, l'invention s'applique également à la mise en place d'un instrument orthopédique de type râpe chirurgicale d'implant orthopédique, notamment une râpe fémorale pour prothèse de hanche. Dans ce cas, la râpe est prévue pour être impactée directement par l'impacteur 10, préférentiellement avec sensiblement la même intensité, sans l'intermédiaire d'un ancillaire de pose, et la surface d'impact correspondante est alors constituée par une surface prévue à cet effet, agencée au niveau d'une extrémité supérieure d'une partie de préhension de la râpe.

Selon l'exemple de réalisation de la figure 1, l'impacteur 10 est équipé d'un capteur de force 12, apte à être positionné sur la surface de frappe 11 destinée à entrer en contact avec la surface d'impact 22 de l'ancillaire de pose 20. Ce capteur de force 12 est destiné à convertir en un signal électrique exploitable, la force d'impaction appliquée sur la surface d'impact 22 de l'ancillaire de pose lors de chaque frappe au moyen de l'impacteur 10 pour l'insertion de l'implant 2 dans la cavité osseuse 3. Selon un exemple de réalisation, le capteur de force peut être constitué de jauges de déformation ou de contrainte disposées au niveau de la surface de frappe 11 de l'outil de percussion 10 et connectées selon un montage approprié. En variante, le capteur de force peut être constitué d'un accéléromètre.

Selon un autre mode de réalisation (non représenté), le capteur de force 12 peut être positionné au niveau de la surface d'impact 22 de l'ancillaire de pose 20 (ou de l'instrument orthopédique lui-même, dans le cas de la mise en place par impaction d'une râpe orthopédique).

Par ailleurs, le dispositif de l'invention comprend des moyens de traitement 30, couplés au capteur de force 12, et agencés, comme il sera décrit plus en détail par la suite, pour évaluer le degré d'insertion de l'implant 2 au niveau de l'os récepteur 3, à partir des signaux de mesure délivrés par le capteur de force 12. Ces moyens de traitement 30, tels qu'un microprocesseur, sont associés à une mémoire 31 destinée à enregistrer les signaux de mesure fournis par le capteur de force 12, les moyens de traitement 30 et la mémoire 31 étant par exemple assemblés sur un circuit imprimé. Les moyens de traitement 30 sont par exemple logés dans un boîtier externe 32. En variante, les moyens de traitement 30 peuvent aussi être intégrés au dispositif qui est équipé avec le capteur de force (impacteur, ancillaire de pose, ou instrument orthopédique dans le cas d'une râpe, selon les différents modes de réalisation). De préférence, la liaison entre le capteur de force 12 et les moyens de traitement 30 est réalisée de manière filaire au moyen d'un câble. Dans une autre variante, la transmission des signaux de mesure acquis par le capteur de force 12 pourrait être faite également au moyen d'une liaison sans fil.

En référence à la figure 1, lors de chaque frappe avec sensiblement la même intensité effectuée au moyen de l'impacteur 10 sur l'ancillaire de pose 20, le capteur de force 12 est conçu pour enregistrer la force d'impaction appliquée en régime dynamique. Plus précisément, lors de chaque frappe successive nécessitée par la mise en place du cotyle prothétique, le capteur de force 12 est apte à fournir un signal de mesure indicatif de la variation temporelle de ladite force d'impaction appliquée. Ainsi, comme illustré à la figure 2, les moyens de traitement 30 disposent pour chaque impact, d'un signal de la variation temporelle de la force d'impaction appliquée.

La figure 2 montre à titre d'exemple six tels signaux de mesure, correspondant à la variation temporelle de la force d'impaction appliquée, lors de différents impacts, respectivement le premier, le deuxième, le troisième, le quatrième, le sixième et le dixième impact effectués au cours d'un test d'insertion du cotyle prothétique dans un os récepteur. Ce test a été mené en utilisant en tant qu'impacteur, une même masse de 3,5 kg lâchée sur la surface d'impact de l'ancillaire de pose depuis une hauteur de 4 cm, de manière à reproduire des impacts de sensiblement la même intensité pendant la durée du test d'insertion. Comme le montre la figure 2, le profil de la variation temporelle de la force d'impaction appliquée par l'impacteur sur l'ancillaire de pose varie fortement en fonction du rang de l'impact. Or, il a pu être démontré que cette variation temporelle de la force d'impaction appliquée lors d'un impact avec sensiblement la même intensité dépend directement de la rigidité du contact entre l'implant et l'os récepteur ainsi que de la force d'arrachement.

Aussi, l'invention se base sur la mesure de la variation temporelle de la force d'impaction entre la surface d'impact de l'ancillaire de pose et la surface de frappe de l'impacteur pour déterminer un indicateur à même de qualifier cet état de rigidité du contact entre l'implant et l'os récepteur et partant, le degré d'insertion de l'implant dans l'os récepteur et la force d'arrachement. En effet, l'augmentation du degré d'insertion de l'implant dans l'os récepteur est lié à l'augmentation de la surface de contact entre l'implant et l'os récepteur, laquelle induit une augmentation de la rigidité du contact entre l'implant et l'os, qui peut avantageusement être révélée par l'analyse de la variation temporelle de la force d'impaction entre l'ancillaire de pose et l'impacteur.

Une première variante permettant de construire un tel indicateur à l'aide des moyens de traitement de l'invention consiste à déterminer la moyenne de la force d'impaction appliquée, lors de chaque impact, calculée sur une fenêtre temporelle de durée programmable positionnée sur le signal de mesure fourni par le capteur, le début de cette fenêtre temporelle étant préférentiellement définie par rapport à un instant correspondant à l'amplitude maximale du signal de mesure. Autrement dit, la fenêtre temporelle permet d'intégrer le signal de mesure correspondant à la variation temporelle de la force d'impaction dans une période de temps programmable.

Selon un premier mode de réalisation, en référence à la figure 2, lors du traitement du signal de mesure résultant de chaque impact, l'instant de début $t_1$ de la fenêtre temporelle F d'intégration, ou limite d'intégration basse, est défini de telle façon qu'il coïncide avec l'instant $t_0$ correspondant à l'amplitude maximale $A_1$ du signal de mesure. En effet, la forme et l'amplitude du pic correspondant à l'amplitude maximale est caractéristique pour chaque signal de mesure acquis, ce qui en fait un point de repère commun facilement reproductible pour la limite d'intégration basse du signal. Selon ce mode de réalisation, la fenêtre temporelle d'intégration débute donc à l'instant correspondant à l'amplitude maximale $A_1$ du signal de mesure. Selon ce premier mode réalisation, l'instant de fin $t_2$ de la fenêtre temporelle F d'intégration du signal de mesure, ou limite d'intégration haute, est choisi de telle manière que la fenêtre temporelle F d'intégration a une durée comprise entre 1,2 ms et 2 ms, avantageusement entre 1,4 ms et 1,8 ms et préférentiellement égale à 1,6 ms.

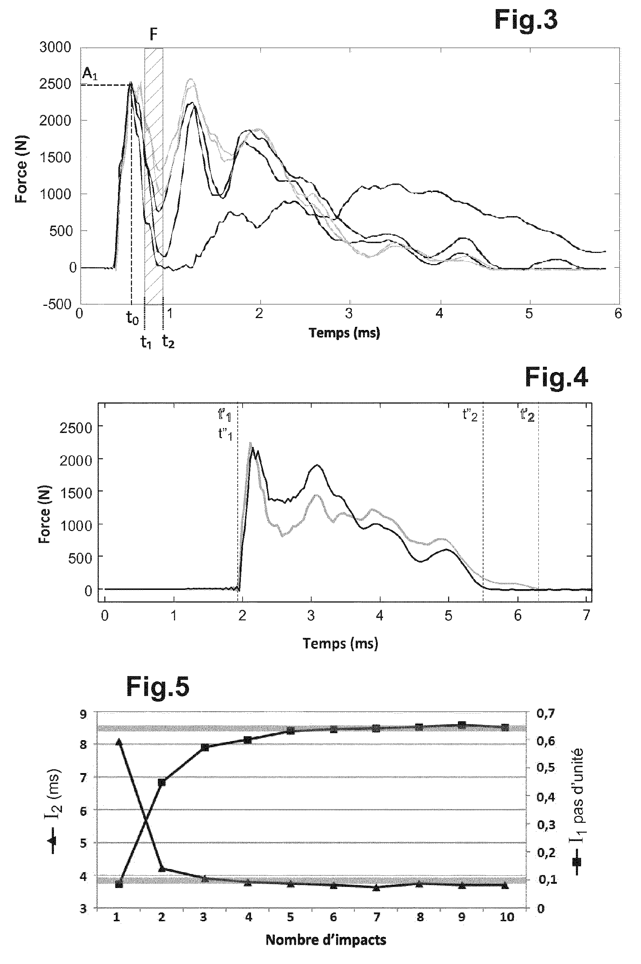

La figure 3 illustre un autre mode de réalisation pour définir la position et la durée de la fenêtre temporelle d'intégration permettant la construction de l'indicateur selon l'invention. La figure 3 illustre les résultats d'une série de tests d'insertion d'un cotyle prothétique dans des os récepteurs de différents diamètres, respectivement 49, 49.5, 50, 50.5 et 51 mm, toujours avec des impacts de sensiblement la même intensité, et la figure 3 illustre plus précisément les différents signaux de variation temporelle de la force d'impaction appliquée par l'impacteur sur l'ancillaire de pose lors du dernier impact de chaque série de test, pour les cinq os récepteurs de différents diamètres, respectivement 49, 49.5, 50, 50.5 et 51 mm.

En particulier, selon cet autre mode de réalisation pour le positionnement de la fenêtre temporelle d'intégration F, l'instant de début $t_1$ de la fenêtre temporelle d'intégration F, autrement dit la limite d'intégration basse du signal de mesure, est défini avec un retard prédéterminé par rapport à l'instant $t_0$ correspondant à l'amplitude maximale $A_1$ du signal de mesure. Par exemple, les limites de la fenêtre temporelle d'intégration F sont choisies comme suit : $t_1 = t_0 + 0.391$ ms and $t_2 = t_0 + 0.527$ms. Il a pu être constaté que le calcul de la moyenne du signal sur une telle fenêtre temporelle d'intégration était appropriée pour l'obtention d'un indicateur fiable et pertinent du degré d'insertion de l'implant et donc de la stabilité de celui-ci. Toutefois, la fenêtre temporelle d'intégration F peut voir sa durée sensiblement modifiée, sans affecter substantiellement les résultats, et peut préférentiellement présenter une durée prédéfinie inférieure ou égale à 10 ms. Elle peut par exemple être comprise entre 0,080 ms et 0,2 ms, avantageusement encore entre 0,098 ms et 1,175 ms. Une fois la fenêtre temporelle d'intégration F positionnée selon l'un ou l'autre des modes de réalisation exposés ci-dessus, l'indicateur $I_1$ représentatif du degré d'insertion de l'implant dans l'os récepteur peut être calculé de la façon suivante :

$$ I_1 = \frac{1}{A_1 . (t_2 - t_1)} \int\limits_{t_1}^{t_2} A(t) . dt $$

où :

A(t) correspond à l'amplitude dudit signal de mesure à l'instant t ;

$A_1$ correspond à l'amplitude maximale dudit signal de mesure ; et

$t_1$ et $t_2$ correspondent respectivement aux instants de début et de fin de ladite fenêtre temporelle F d'intégration.

Avantageusement, la valeur de cet indicateur est corrélée à la force d'arrachement, de sorte que sa valeur ainsi calculée permet de fournir une mesure de la force d'arrachement de l'implant et donc de la stabilité mécanique de l'implant.

Selon une seconde variante de réalisation, un indicateur $I_2$ représentatif du degré d'insertion de l'implant dans l'os récepteur peut également être déterminé par les moyens de traitement comme étant la durée d'impact mesurée à partir de la variation temporelle de la force d'impaction appliquée pour chaque impact. En effet, une augmentation de la surface de contact entre l'implant et l'os au cours de l'insertion de l'implant dans l'os induit une augmentation de la rigidité du contact entre l'implant et l'os, ce qui se traduit par une diminution de la durée d'impact lors des impacts successifs. Autrement dit, la durée d'impact, pendant laquelle l'effort est transmis de l'outil de percussion vers l'ancillaire de pose, est révélatrice de la rigidité du contact os-implant et donc du degré d'insertion de l'implant dans l'os récepteur, dont dépend la stabilité primaire de l'implant.

Aussi, cette variante de réalisation du calcul de l'indicateur $I_2$ repose sur le constat suivant lequel la durée d'impact, lors de chaque impact, est à même de constituer une information pertinente permettant de caractériser de manière fiable et précise les propriétés mécaniques de l'interface os-implant et partant, la rigidité du contact os-implant.

Ainsi, selon cette variante, lors de chaque impact pendant l'insertion de l'implant (avec sensiblement la même intensité), le capteur de force 12 fournit un signal de mesure indicatif de la variation temporelle de la force d'impaction appliquée et la durée de chaque impact est calculée à partir de la variation temporelle de la force d'impaction appliquée pour construire un indicateur $I_2$ représentatif du degré d'insertion de l'implant comme expliqué plus haut. La figure 4 illustre deux tels signaux enregistrés par le capteur de force pendant deux impacts différents, dans les conditions de test exposées en référence à la figure 2. Le signal en traits pointillés correspond au signal recueilli lors du troisième impact et le signal en trait plein correspond au signal recueilli lors du sixième impact. Selon un mode de réalisation, les instants t'1, t"1 (respectivement les instants t'2, t"2) correspondant au début (respectivement la fin) de l'impact pour chacun de ces signaux, ont été calculés en déterminant la limite de temps inférieure (respectivement la limite de temps supérieure) pour chaque impact où l'amplitude de la force d'impaction appliquée est supérieure à une valeur seuil prédéfinie, par exemple 30N. Il en résulte alors l'indicateur $I_2$ correspondant à la durée de la fenêtre temporelle bornée par ces deux limites de temps inférieure et supérieure, calculé comme suit : $I_2$ = t'2 - t'1, soit $I_2$ = 4,5 ms pour le signal correspondant au troisième impact et $I_2$ = t"2 - t"1, soit $I_2$ = 3,5 ms pour le signal correspondant au sixième impact.

Quelle que soit la variante de réalisation retenue pour la construction de l'indicateur $I_1$ ou $I_2$ représentatif du degré d'insertion de l'implant dans l'os récepteur, les moyens de traitement 30 sont alors conçus pour contrôler l'évolution de l'indicateur en fonction du nombre d'impacts, afin d'évaluer, à partir de cette évolution, le degré d'insertion de l'implant dans l'os et par conséquent, la stabilité primaire de l'implant.

Les signaux montrés à la figure 2 correspondent aux signaux de rang 1, 2, 3, 4, 6 et 10 recueillis au cours du test d'insertion du cotyle prothétique dans un os récepteur dans les conditions exposées plus haut en référence à la figure 2. La figure 5 représente les variations correspondantes des deux indicateurs $I_1$ et $I_2$ en fonction du nombre d'impacts. On constate que l'indicateur $I_1$ augmente au cours des six premières frappes puis atteint un palier à partir du sixième impact où sa valeur n'évolue sensiblement plus. Concernant l'indicateur $I_2$ correspondant à la durée d'impact, on constate qu'il ne varie que très faiblement après le troisième impact, tandis que la forme globale des signaux continue d'évoluer jusqu'au sixième impact. Après le sixième impact, les signaux sont quasiment superposés, conduisant à des valeurs sensiblement constantes pour les deux indicateurs, respectivement $I_1$ et $I_2$.

Aussi, afin de déterminer le degré d'insertion de l'implant dans l'os récepteur, les moyens de traitement comprennent des moyens de contrôle de la convergence de l'indicateur $I_1$ ou $I_2$ vers une valeur stationnaire en fonction du nombre d'impacts. Aussi, les moyens de traitement de l'invention sont par exemple conçus pour détecter lorsque l'indicateur atteint un palier de saturation, où sa valeur n'évolue sensiblement plus, indiquant par là même que le degré d'insertion de l'implant dans l'os récepteur est suffisant. En effet, l'atteinte par l'indicateur d'un palier sensiblement stable à partir d'un certain nombre d'impacts signifie que la rigidité du contact entre l'implant et l'os récepteur au cours du processus d'insertion ne varie plus, moyennant quoi le degré d'insertion est considéré comme optimal ou, en tous les cas, comme suffisant. La valeur de l'indicateur, corrélée à la force d'arrachement, est également prise en compte par les moyens de traitements pour déterminer la stabilité implantaire.

Ce comportement peut s'expliquer par le fait que l'implant s'enfonce progressivement dans l'os récepteur lors d'une première phase des impacts. Ainsi, la surface d'os au contact de l'instrument orthopédique augmente, ce qui induit une augmentation de la rigidité du contact se traduisant par une évolution de l'indicateur, en particulier par une augmentation de ce dernier suivant la variante où il mesure, à partir de la variation temporelle de la force appliquée, la moyenne de la force sur une fenêtre temporelle d'intégration prédéfinie, ou par une diminution de ce dernier suivant la variante où il mesure, à partir de la variation temporelle de la force appliquée, la durée d'impact. Au bout d'un certain nombre d'impacts (six selon l'exemple de la figure 5), l'indicateur atteint un palier car l'implant est complètement enfoncé dans l'os et la surface de contact entre l'os et l'instrument orthopédique ne peut donc plus augmenter, ce qui explique le palier atteint par l'indicateur selon l'invention. Dit autrement, le principe de mesure de l'indicateur selon l'invention se base sur une estimation de la rigidité du contact os-instrument orthopédique, par quoi le degré d'insertion de l'implant dans l'os récepteur peut être évalué.

D'autres approches que celle qui vient d'être décrite pourraient également être envisagées afin d'évaluer le degré d'insertion à partir des signaux de mesure enregistrés par le capteur de force. Néanmoins, quelle que soit l'approche retenue, le principe de base de l'invention, sur lequel repose le traitement des signaux de mesure acquis par le capteur de force pour l'évaluation du degré d'insertion, consiste à utiliser la variation de la force d'impaction appliquée en fonction du temps pour chaque frappe ou bien, en variante, à utiliser la transformée de Fourier de la variation de la force d'impaction en fonction du temps (domaine fréquentiel).

Suivant ce qui précède, on considère donc que le degré d'insertion de l'implant dans l'os récepteur est suffisant dès que les moyens de traitement 30 détectent que l'indicateur $I_1$ ou $I_2$ a atteint un palier de saturation stable, signifiant que la rigidité du contact os-implant ne varie plus.

En variante, on peut aussi considérer que le degré d'insertion de l'implant est suffisant lorsque les moyens de traitement 30 détectent que l'indicateur a atteint une valeur de seuil prédéterminée, à l'aide d'abaques.

Les moyens de traitement peuvent en outre être couplés à des moyens 33 d'alerte sonore et/ou visuelle, aptes à fournir une indication visuelle et/ou sonore au chirurgien lorsque la stabilité primaire évaluée par les moyens de traitement est jugée suffisante, autrement dit, dès que l'indicateur atteint un palier. Dès lors, le chirurgien dispose en temps réel d'une information fiable lui indiquant qu'il a atteint un degré d'insertion suffisant de l'implant dans l'os récepteur et qu'il n'est plus nécessaire de continuer à impacter l'implant, lui évitant ainsi tout risque de provoquer une fracture de l'os récepteur.

Une application du dispositif et de la méthode qui viennent d'être décrits concerne la pose d'implant de prothèse de hanche, avec l'exemple particulier de la pose du cotyle prothétique. Bien entendu, toujours dans le cadre de la pose de prothèse totale de hanche, l'invention pourrait aussi s'appliquer à la pose de la tige fémorale. En outre, d'autres applications de la présente invention dans le cadre de la chirurgie orthopédique sont également envisageables, qui concernent la pose d'implants pour des prothèses du genou, d'épaule ou de cheville et, de manière générale, la mise en place de tout type d'instrument orthopédique nécessitant d'être inséré dans un os récepteur par impaction.

**Revendications**

1. Dispositif (1) d'assistance à la mise en place d'un instrument orthopédique (2) destiné à être inséré au niveau d'un os récepteur (3) par impaction, ledit dispositif comprenant un impacteur (10) comportant au moins une surface de frappe (11) adaptée pour venir impacter une surface d'impact couplée audit instrument orthopédique pour appliquer une force d'impaction audit instrument orthopédique (2), ledit dispositif comprenant un capteur de force (12) apte à mesurer, lors de chaque impact, ladite force d'impaction appliquée par l'impacteur (10) sur ladite surface d'impact **caractérisé en ce que** le capteur de force est apte à fournir un signal de mesure indicatif de la variation temporelle de ladite force d'impaction appliquée, ledit capteur de force (12) étant relié à des moyens de traitement (30) conçus pour déterminer, à partir de la variation temporelle de ladite force d'impaction fournie pour chaque impact successivement, un indicateur ($I_1$) représentatif du degré d'insertion dudit instrument orthopédique (2) dans l'os récepteur (3).

2. Dispositif selon la revendication 1, **caractérisé en ce que** ledit indicateur ($I_1$) est déterminé par lesdits moyens de traitement comme étant la moyenne de ladite force d'impaction appliquée, calculée sur une fenêtre temporelle (F) de durée programmable positionnée sur ledit signal de mesure, le début de ladite fenêtre temporelle étant définie par rapport à un instant ($t_0$) correspondant à l'amplitude maximale ($A_1$) dudit signal de mesure.

3. Dispositif selon la revendication 2, **caractérisé en ce que** l'instant de début ($t_1$) de ladite fenêtre temporelle (F) coïncide avec l'instant ($t_0$) correspondant à l'amplitude maximale dudit signal de mesure.

4. Dispositif selon la revendication 2 ou 3, **caractérisé en ce que** ladite fenêtre temporelle (F) a une durée comprise entre 1,2 ms et 2 ms.

5. Dispositif selon la revendication 2, **caractérisé en ce que** l'instant de début ($t_1$) de ladite fenêtre temporelle (F) est défini avec un retard prédéterminé par rapport à l'instant ($t_0$) correspondant à l'amplitude maximale ($A_1$) dudit signal de mesure.

6. Dispositif selon la revendication 5, **caractérisé en ce que** ladite fenêtre temporelle (F) a une durée prédéfinie inférieure ou égale à 10 ms.

7. Dispositif selon l'une quelconque des revendications 2 à 6, **caractérisé en ce que** ledit indicateur ($I_1$) est calculée de la façon suivante :

$$I_1 = \frac{1}{A_1.(t_2 - t_1)}\int_{t_1}^{t_2} A(t).dt$$

où :

A(t) correspond à l'amplitude dudit signal de mesure à l'instant t ;

$A_1$ correspond à l'amplitude maximale dudit signal de mesure ; et

$t_1$ et $t_2$ correspondent respectivement aux instants de début et de fin de ladite fenêtre temporelle.

8. Dispositif selon l'une quelconque des revendications précédentes, **caractérisé en ce que** lesdits moyens de traitement (30) comprennent des moyens de contrôle de la convergence dudit indicateur vers une valeur stationnaire dudit indicateur en fonction du nombre d'impacts, de manière à évaluer ledit degré d'insertion dudit instrument orthopédique (2) dans l'os récepteur (3).

9. Dispositif selon l'une quelconque des revendications précédentes, **caractérisé en ce que** ledit instrument orthopédique (2) est constitué d'un implant pour prothèse orthopédique, ledit impacteur (10) agissant pour impacter ledit implant en coopération avec un ancillaire de pose (20) consistant en un manche rigide **(21)** de manipulation, pourvu dans sa partie distale, d'une tête de préhension (26) dudit implant (2) et, dans sa partie proximale, de ladite surface (22) d'impact.

10. Dispositif selon l'une quelconque des revendications 1 à 8, **caractérisé en ce que** ledit instrument orthopédique (2) est constitué d'une râpe chirurgicale d'implant orthopédique, notamment une râpe fémorale pour prothèse de hanche, comportant une partie de préhension pourvue à son extrémité supérieure de ladite surface d'impact.

11. Dispositif selon l'une quelconque des revendications précédentes, **caractérisé en ce que** ledit capteur de force (12) est monté sur ladite surface de frappe (11) dudit impacteur (10).

12. Dispositif selon l'une quelconque des revendications 1 à 10, **caractérisé en ce que** ledit capteur de force (12) est monté sur ladite surface d'impact.

13. Dispositif selon l'une quelconque des revendications précédentes, **caractérisé en ce que** ledit capteur de force est constitué de jauges de déformations.

14. Dispositif selon l'une quelconque des revendications 1 à 12, **caractérisé en ce que** ledit capteur de force est constitué d'un accéléromètre.

**Patentansprüche**

1. Hilfsvorrichtung (1) zum Anbringen eines orthopädischen Instruments (2), das dazu vorgesehen ist, auf Höhe eines Empfängerknochens (3) durch Eintreiben angebracht zu werden, wobei die Vorrichtung einen Hammer (10) umfasst, der mindestens eine Schlagfläche umfasst (11), die angepasst ist, auf eine Prallfläche aufzuprallen, die mit dem orthopädischen Instrument gekoppelt ist, um eine Schlagkraft auf das orthopädische Instrument (2) anzuwenden, wobei die Vorrichtung einen Kraftsensor (12) umfasst, der geeignet ist, bei jedem Aufprall die Schlagkraft zu messen, die von dem Hammer (10) auf die Prallfläche aufgebracht wird, **dadurch gekennzeichnet, dass** der Kraftsensor geeignet ist, ein Messsignal zu liefern, das die zeitliche Veränderung der aufgebrachten Schlagkraft anzeigt, wobei der Kraftsensor (12) an Verarbeitungsmittel (30) angeschlossen ist, die ausgestaltet sind, anhand der aufeinanderfolgend für jeden Aufprall gelieferten zeitlichen Veränderung der Schlagkraft eine Anzeige ($I_1$) zu bestimmen, die den Grad der Insertion des orthopädischen Instruments (2) in den Empfängerknochen (3) darstellt.

2. Vorrichtung nach Anspruch 1, **dadurch gekennzeichnet, dass** die Anzeige ($I_1$) durch die Verarbeitungsmittel bestimmt wird als Mittelwert der aufgebrachten Schlagkraft, der über ein Zeitfenster (F) berechnet wird, wobei das Zeitfenster (F) von programmierbarer Dauer ist und über dem Messsignal positioniert ist, wobei der Beginn des Zeitfensters bestimmt wird in Bezug zu einem Moment ($t_0$), der der maximalen Amplitude ($A_1$) des Messsignals entspricht.

3. Vorrichtung nach Anspruch 2, **dadurch gekennzeichnet, dass** der Moment des Beginns ($t_1$) des Zeitfensters (F) mit dem Moment ($t_0$) zusammenfällt, der der maximalen Amplitude des Messsignals entspricht.

4. Vorrichtung nach Anspruch 2 oder 3, **dadurch gekennzeichnet, dass** das Zeitfenster (F) eine Dauer zwischen 1,2 ms und 2 ms aufweist.

5. Vorrichtung nach Anspruch 2, **dadurch gekennzeichnet, dass** das der Moment des Beginns (t1) des Zeitfensters (F) bestimmt wird mit einer vorbestimmten Verzögerung in Bezug zu dem Moment ($t_0$), der der maximalen Amplitude ($A_1$) des Messsignals entspricht.

**6.** Vorrichtung nach Anspruch 5, **dadurch gekennzeichnet, dass** das Zeitfenster (F) eine vorbestimmte Dauer aufweist, die weniger oder gleich 10 ms ist.

**7.** Vorrichtung nach einem der Ansprüche 2 bis 6, **dadurch gekennzeichnet, dass** die Anzeige ($I_1$) wie folgt berechnet wird:

$$I_1 = \frac{1}{A_1 \cdot (t_2 - t_1)} \int_{t_1}^{t_2} A(t).dt$$

wobei:

$A(t)$ der Amplitude des Messsignals zum Moment t entspricht;
$A_1$ der maximalen Amplitude des Messignals entspricht; und
$t_1$ und $t_2$ jeweils dem Moment des Beginns und des Endes des Zeitfensters entsprechen.

**8.** Vorrichtung nach einem der vorhergehenden Ansprüche, **dadurch gekennzeichnet, dass** die Verarbeitungsmittel (30) Mittel zur Kontrolle der Übereinstimmung der Anzeige mit einem statischen Wert der Anzeige in Abhängigkeit von der Aufprallanzahl umfassen, um den Grad der Insertion des orthopädischen Instruments (2) in den Empfängerknochen (3) zu bestimmen.

**9.** Vorrichtung nach einem der vorhergehenden Ansprüche, **dadurch gekennzeichnet, dass** das orthopädische Instrument (2) aus einem orthopädischen Prothesenimplantat besteht, wobei der Hammer (10) zum Eintreiben des Implantats mit einem Hilfsinstrument zum Einsetzen (20) zusammenwirkt, das aus einem starren Betätigungsstiel (21) besteht, der in seinem distalen Bereich mit einem Greifkopf (26) des Implantats (2) und in seinem proximalen Bereich mit der Prallfläche (22) versehen ist.

**10.** Vorrichtung nach einem der Ansprüche 1 bis 8, **dadurch gekennzeichnet, dass** das orthopädische Instrument (2) aus einer chirurgischen Raspel eines orthopädisches Implantats besteht, insbesondere einer Oberschenkelraspel für eine Hüftprothese, die einen Griffbereich umfasst, der an ihrem Ende oberhalb der Prallfläche vorgesehen ist.

**11.** Vorrichtung nach einem der vorhergehenden Ansprüche, **dadurch gekennzeichnet, dass** der Kraftsensor (12) auf der Schlagfläche (11) des Hammers (10) angebracht ist.

**12.** Vorrichtung nach einem der Ansprüche 1 bis 10, **dadurch gekennzeichnet, dass** der Kraftsensor (12) auf der Prallfläche angebracht ist.

**13.** Vorrichtung nach einem der vorhergehenden Ansprüche, **dadurch gekennzeichnet, dass** der Kraftsensor aus Dehnungsmessstreifen besteht.

**14.** Vorrichtung nach einem der Ansprüche 1 bis 12, **dadurch gekennzeichnet, dass** der Kraftsensor aus einem Beschleunigungsmesser besteht.

**Claims**

**1.** A device (1) for assisting the placement of an orthopedic instrument (2) intended to be inserted by impacting a receiving bone (3), said device comprising an impacter (10) including at least a striking surface (11) adapted to impact an impact surface coupled to said orthopedic instrument to apply an impacting force to said orthopedic instrument (2), said device comprising a force sensor (12) able to measure, upon each impact, said impacting force applied by the impacter (10) on said impact surface,
**characterized in that** the force sensor is able to provide a measurement signal indicative of the temporal variation of said applied impacting force, said force sensor (12) being connected to processing means (30) designed to determine, from the temporal variation of said impacting force successively provided for each impact, an indicator ($I_1$) representative of the degree of insertion of said orthopedic instrument (2) within the receiving bone (3).

**2.** The device according to claim 1, **characterized in that** said indicator ($I_1$) is determined by said processing means as being the average of said applied impacting force, calculated on a temporal window (F) of programmable duration positioned on said measurement signal, the start of said temporal window being defined with respect to a time ($t_0$) corresponding to the maximum amplitude ($A_1$) of said measurement signal.

**3.** The device according to claim 2, **characterized in that** the starting time ($t_1$) of said temporal window (F) coincides with the time ($t_0$) corresponding to the maximum amplitude of said measurement signal.

**4.** The device according to claim 2 or 3, **characterized in that** said temporal window (F) has a duration ranging between 1,2 ms and 2 ms.

**5.** The device according to claim 2, **characterized in that** the starting time ($t_1$) of said temporal window (F) is defined with a predetermined delay with respect to the time ($t_0$) corresponding to the maximum amplitude ($A_1$) of said measurement signal.

**6.** The device according to claim 5, **characterized in that** said temporal window (F) has a predefined duration of less than or equal to 10 ms.

7. The device according to any of claims 2 to 6, **characterized in that** said indicator ($I_1$) is calculated in the following way:

$$I_1 = \frac{1}{A_1.(t_2 - t_1)} \int_{t_1}^{t_2} A(t).dt$$

where:

A(t) corresponds to the amplitude of said measurement signal at the time t ;
$A_1$ correspond to the maximum amplitude of said measurement signal; and
$t_1$ and $t_2$ correspond respectively to the starting and ending times of said temporal window.

8. The device according to any of the preceding claims, **characterized in that** said processing means (30) comprise means for monitoring the convergence of said indicator toward a steady value of said indicator as a function of the number of impacts, so as to evaluate said degree of insertion of said orthopedic instrument (2) into the receiving bone (3).

9. The device according to any of the preceding claims, **characterized in that** said orthopedic instrument (2) consists of an implant for an orthopedic prosthesis, said impacter (10) acting to impact said implant in cooperation with a placement ancillary (20) consisting in a handling rigid stick (21), provided, in its distal part, with a gripping head (26) for said implant (2) and, in its proximal part, with said impact surface (22).

10. The device according to any of claims 1 to 8, **characterized in that** said orthopedic instrument (2) consists of an orthopedic implant surgical rasp, particularly a femoral rasp for hip prosthesis, including a gripping part provided at its upper end with said impact surface.

11. The device according to any of the preceding claims, **characterized in that** said force sensor (12) is mounted on said striking surface (11) of said impacter (10).

12. The device according to any of claims 1 to 10, **characterized in that** said force sensor (12) is mounted on said impact surface.

13. The device according to any of the preceding claims, **characterized in that** said force sensor consists of strain gages.

14. The device according to any of claims 1 to 12, **characterized in that** said force sensor consists of an accelerometer.

**Fig.1**

**Fig.2**

**Fig.3**

**Fig.4**

**Fig.5**

**RÉFÉRENCES CITÉES DANS LA DESCRIPTION**

**Documents brevets cités dans la description**

- EP 1433445 A
- US 2005101962 A